# EUROPEAN PATENT APPLICATION

(11) **EP 4 088 713 A1**
(43) Date of publication of application: **16.11.2022**
(21) Application number: 21460024.9
(22) Date of filing: 10.05.2021
(51) Int. Cl.: A61K 9/00, A61K 47/02, A61K 47/18, A61K 47/40, A61K 9/08, A61K 31/46, A61P 27/08

(54) **OPHTHALMIC PHARMACEUTICAL COMPOSITION COMPRISING ATROPINE**

(71) Applicant: Warszawskie Zaklady Farmaceutyczne Polfa S.A., 01-207 Warszawa (PL)
(72) Inventor: PIETRZAK, Tomasz, 95-020 Wisniowa Gora (PL); CEBULSKA, Ewelina, 05-650 Chynow (PL); LUCZYNSKI, Pawel, 02-776 Warszawa (PL); MAGNUSZEWSKA, Monika, 05-806 Nowa Wies Warszawska (PL)
(74) Representative: Kondrat, Mariusz

(57) **Abstract**

The present invention is related to a preservative free ophthalmic low concentration atropine sulfate composition containing atropine sulfate in an amount from about 0.005 w/w% to about 0.05 w/w%, a tonicity agent, water and a pH adjuster, having a pH from about 4.0 to about 5.5. Optionally this composition can also contain other excipients such as a chelating agent, a complexing agent, and/or a viscosity agent. The invention also relates to a process for the preparation of this composition and its use in the treatment and/or prevention of myopia, preferably in a paediatric population.

## Description

### Technical field

The present invention relates to a preservative free ophthalmic pharmaceutical composition comprising atropine or a pharmaceutically acceptable salt thereof as active ingredient.

### State of the art

Myopia (or short-sightedness) is an ophthalmic condition that leads to blurred long-distance vision, generally characterized by a refractive error of -0.5 or -1 diopters. Overall, it has been estimated that currently 1.4 billion people in the world are myopic (22.9% of the population), and crude estimations suggest that, by 2050, there will be 4.7 billion people affected (nearly 50% of the world population) (Ophthalmology 2016, 123, 1036-1042). Pharmacological treatments have however shown some very promising potential. Of these, mydriatic agents such as atropine or tropicamide gained interest (Asia Pac. J. Ophthalmol. 2018, 7, 405-414), with atropine being the most studied. Its biological action has been surmised as involving a complex interplay with receptors on different ocular tissues at multiple levels, leading to a decrease in change in the cycloplegic refraction and axial length elongation (Pharmaceutics 2020, 12, 781).

Several trials have been performed to prove safety and effectiveness at different concentrations of atropine in controlling myopia progression. Initially tested at 1% concentration during the ATOM1 trial, atropine eyedrops were proven to control myopic progression effectively but caused important visual side effects resulting from cycloplegia and mydriasis (Ophthalmology 2006, 113, 2285-2291). Since then, several clinical trials have evaluated the safety and efficiency of atropine eye drops at lower concentrations. The ATOM2 trial studied myopia progression in 400 children 2 years after treatment with 0.01%, 0.1%, and 0.5% and found the 0.01% concentration to be the concentration causing the least side effects with comparable efficacy in controlling myopia progression (Ophthalmology 2012, 119, 347-354; Ophthalmology 2016, 123, 391-399). Very recently, the LAMP phase 2 trial report confirmed that concentrations ranging from 0.01% to 0.05% were well tolerated in 383 children after two years of treatment, with patients experiencing rare and mild side effects (Ophthalmology 2019, 126, 113-124), even if the need for photochromatic glasses was higher than 30% for treated patients.

Despite all this recent and very favourable clinical data, there is still currently no commercially available low dose atropine ophthalmic compositions.

The catabolic degradation of aqueous atropine is well described. Instability is due to either hydrolysis, which yields tropine and tropic acid, or dehydration, which yields apoatropine. The stability of aqueous atropine sulfate is dependent on several variables. It is well established that stability is enhanced in acid solution. Ideal storage pH for aqueous atropine sulfate solution ranges approximately between 3 and 4 (Lund W, Waaler T. The kinetics of atropine and apoatropine in aqueous solutions. Acta Chem Scand. 1968; 22:3085-97). However, eye pain and stinging may occur upon the instillation of acidic atropine sulfate ophthalmic solution. This could jeopardize the compliance of a long treatment as it is the case for the treatment of myopia in children. Therefore, atropine sulfate ophthalmic solutions with a more acidic pH need to be formulated to allow the eye to adjust the pH to a physiological value of 7.4 as quick as possible.

The international patent application WO2017204262-A2 discloses an aqueous composition comprising 0.001 - 0.1 % (w/v) atropine or a salt thereof, a buffer (I) and a water-soluble polymer, which is at a pH range of 6 or lower. It is described that the buffer (I) is at least one selected from the group consisting of a phosphate buffer, an aminocarboxylate buffer, a carbonate buffer, an acetate buffer, a tartrate buffer, a borate buffer, and trometamol. Preferred buffer (I) is phosphate buffer. This aqueous composition further comprises a buffer (II), preferably citrate buffer. It is stated that this aqueous composition has a potent action for inhibiting the elongation of eye axial length and improving the refractive error without exacerbating the mydriatic action of atropine. Additionally, a non-ionic tonicity agent is needed to inhibit the debasement over time of the viscosity given by the water-soluble polymer and additionally maintain the stability of atropine or a salt thereof. The stability of atropine was studied by measuring the amounts of tropic acid. The study showed an increase in tropic acid in only four weeks, and this is less than desirable.

The international patent application WO2015200361-A1 disclosed an ophthalmic composition comprising from about 0.001 wt% to about 0.05 wt% of atropine or atropine sulfate, water and a buffering agent to provide a pH from about 3.8 to about 7.5. Several ophthalmic water solutions are described in the examples comprising either citric acid or acetic acid. Compositions comprising citric acid are described to have a pH 5.8, 6.4 and 6.8. Compositions comprising acetic acid are described to have a pH of 4.2 and 4.8. The stability of these low concentration ophthalmic compositions of atropine is addressed in relation to the use of deuterated water. It is disclosed that deuterated water has lowered buffering capacity than H2O. Therefore, ophthalmic formulations or solutions formulated in deuterated water allow them to reach physiological pH when administered into the eye at a faster rate than compared to an equivalent ophthalmic formulation or solution formulated in H₂O.

The international patent application WO2018209051-A1 discloses a liquid storage-stable low-dose ophthalmic composition comprising equal or less than 0.05 wt% atropine or a pharmaceutically acceptable salts thereof, a buffer, a tonicity agent and a viscosity modifier. These compositions are described to be stable with a pH near to a neutral pH between 5.0 and 6.0 and using buffering systems with a concentration from about 10 mM to about 75mM. These compositions are free of preservative. Several viscosity modifiers are cited, and cellulosic viscosity modifiers are said to be preferred.
There it is disclosed that the stability of the atropine in these compositions is bounded by the use of a specific low concentration of buffering system at also a specified pH range in combination with the other cited components, such as the presence of a viscosity modifier. Specifically, it is disclosed that once the buffer concentration was adjusted to 10 mM-75 mM at a pH of between 5.0-6.0, the stability of the atropine increases with less formation of tropic acid (a byproduct of atropine hydrolysis). Several examples are disclosed in WO2018209051-A1 in tables 1-3. All these examples disclose aqueous atropine sulfate compositions with a viscosity modifier, specifically hypromellose 2910. The buffer cited in the examples is always monobasic and dibasic sodium phosphate buffer.

The international patent application WO2020219707-A1 discloses topical ophthalmic compositions comprising a list of active components wherein atropine is among them and further a buffer, having a pH of about 3.0 to about 5.5 and additionally, these compositions do not contain a viscosity-enhancing component. The concentration of atropine is described from 0.01 to 2% w/w. A suitable buffer is described to stabilize the stored compositions by maintaining the compositions at a low pH (e.g., pH of about 3.0 to 7.4, or about 3.0 to about 6.0, or about 3.0 to about 5.5), but that quickly equilibrates to a physiological pH (i.e., the pH of the tear film and/or ocular surface, or a pH of about 7.0) when the compositions are administered to the surface of an eye. These topical ophthalmic compositions may contain any one of the buffers listed in table 2: boric acid buffer, borate buffer, borate citrate buffer and lactate buffer. Optionally these compositions also comprise a secondary buffering agent that includes citrate buffer and acetate buffer (Table 3). These compositions can further comprise a preservative.

Several examples are disclosed in WO2020219707-A1. Example 2 discloses Formulations 1-4 comprising 1% w/w of atropine sulfate with boric acid as buffer and sodium citrate dihydrate as a secondary buffering agent (Formulation 1-2) or with sodium lactate as buffer and sodium citrate dihydrate as a secondary buffering agent (Formulation 3-4). All Formulations 1-4 were adjusted to pH 6.0. Example 4 discloses 1% atropine aqueous formulation (Formulation C). Boric acid is the buffer used and further sodium citrate dihydrate as a secondary buffering agent. The pH is adjusted to 4.35. The stability of this formulation is not disclosed. Example 5 discloses 3 formulations (Formulation I-III) with 0.03% w/w of atropine sulfate and also having boric acid and sodium citrate dihydrate as a mixture of buffers. These formulations were described to have an initial (T₀) pH of 4.74, 4.80 and 4.79, respectively. In all the cases, the pH was not stable and gradually decreased along the time, reaching 4.45. 4.56 and 4.31 after 6 months at 25°C/40%RH, respectively. The stability study results suggest that the mixture of both buffers along with the presence of glycine or benzalkonium chloride do not provide sufficient stability for 0.03% atropine aqueous formulations.

The international patent application WO9526711 describes ophthalmic composition in the form of a topical solutions consisting of an ophthalmologically active agent, an ion sensitive, a hydrophilic polymer in an amount of 0.004 to 1.5 % by weight, at least one salt selected from the group of inorganic salts and buffers in a total amount from 0.01 to 2.0 % by weight. Optionally, these compositions can also contain a wetting agent, a preservative and a pH regulating agent. It is described that these solutions exhibit a pH of 4.0 to 8.0, preferably 6.5 to 8.0 and a viscosity of less than 1000 mPas. Several typical examples of basic drug molecules useful in eye therapy are described with atropine being one of them. Timolol or salt thereof is described as preferred, and all the examples are related to timolol. The stability of these eye drops is not addressed.

Despite the ophthalmic solutions available so far in the art, there is still the need to provide further ophthalmic formulations comprising low concentration of atropine sulfate which remain stable, i.e., substantially resistant against hydrolysis, with an assay of atropine within an acceptable range and free of degradation products, over time and are suitable for use for the treatment of myopia, including also paediatric population. Furthermore, these ophthalmic formulations are easy to be manufactured through a low cost and automatized process.

### Object of the invention

The object of the present invention is preservative free ophthalmic low concentration atropine sulfate composition.

Another aspect of the invention is a pharmaceutical dosage form comprising said ophthalmic composition.

Another aspect of the invention is a process for the preparation of such ophthalmic composition.

Another aspect of the invention is the preservative free ophthalmic low concentration atropine sulfate composition for use in the treatment for use in therapy, preferably, for the treatment and/or prevention of myopia, preferably in paediatric population.

### Detailed description of the invention

The object of the present invention is a preservative free ophthalmic low concentration atropine sulfate composition consisting of:
a) atropine sulfate in an amount from about 0.005 w/w% to about 0.05 w/w%
b) a tonicity agent
c) water
d) a pH adjuster, preferably hydrochloric acid
e) optionally a chelating agent, preferably EDTA
f) optionally a complexing agent, and
g) optionally a viscosity agent
characterized in that it has a pH from about 4.0 to about 5.5.

The authors of the present invention have developed a preservative free ophthalmic low concentration atropine sulfate composition consisting of atropine sulfate, a tonicity agent, a pH adjuster and water wherein the required stability of the atropine in ophthalmic compositions is achieved as well as the comfort of the eye upon the instillation of the drop in the eye is guaranteed by adjusting the pH of the solution from 4.0 to 5.5.

Surprisingly, the authors of the present invention have found that the preservative free ophthalmic low concentration atropine sulfate composition consisting of atropine sulfate, a tonicity agent, a pH adjuster and water fulfils the required stability of the atropine in ophthalmic compositions as well as it guarantees the comfort of the eye upon the instillation of the drop in the eye adjusting the pH of the solution from about 4.0 to about 5.5 without the use of any buffer. The presence of a pH adjuster both significantly reduces the level of impurities and maintains the level of atropine within a tolerance limit during shelf life of a pharmaceutical composition. It also guarantees the comfort of the eye upon the instillation, as it allows the eye to adjust the pH to a physiological value of 7.4 as quick as possible. Additionally, this technical effect is overly found when the preservative free ophthalmic low concentration atropine sulfate composition consisting of atropine sulfate, a tonicity agent, pH adjuster and water and having a pH from about 4.0 to about 5.5 also contains a chelating agent. The combination in this ophthalmic solution of the presence of a chelating agent together with the adjustment of the pH from about 4.0 to about 5.5 assures the stability of this unbuffered ophthalmic composition as well as guarantees the comfort of the eye upon instillation of an ophthalmic composition having a pH from about 4.0 to about 5.5. The presence of a chelating agent, preferably EDTA, in this unbuffered formulation increases the stability of atropine sulfate, and hence decreases the concentration of impurities, which are responsible for both fluctuations of the pH value as well as irritating effect of the composition. Moreover, EDTA substantially increases the corneal permeability of atropine, thus improving the effectiveness of the drug.

Along the present description, as well as in the claims, the singular expressions, generally preceded by the articles "a", "an" or "the", are meant to include also the plural forms, unless the context clearly indicates otherwise. Furthermore, if it is not stated on the contrary, numeric values preceded by the term "about" are meant to include the exact stated value and also a certain variation around such value, namely a variation of ±5% of the stated amount. Numeric ranges defined by lower and upper endpoints are meant to include also said stated endpoints. The percentages disclosed are as "w/v%" meaning weight by volume percentages or as w/w% meaning weight by weight percentages. Moreover, it is noted that weight percentages of atropine sulfate provided herein are based on atropine sulfate monohydrate.

### Atropine

Atropine occurs naturally in a number of plants of the nightshade family, including deadly nightshade, Jimson weed, and mandrake. It was first isolated in 1833 (Neurological aspects of substance abuse (2 ed.), 309-315 (2004). It is on the World Health Organization's List of Essential Medicines, the most effective and safe medicines needed in a health system. The chemical structure of atropine is shown in Figure I.

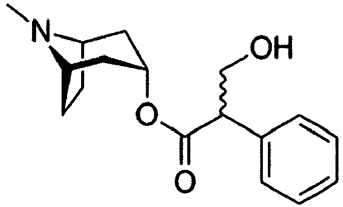

Atropine is currently the most effective therapy for myopia control. Recent clinical trials demonstrated low-dose atropine eye drops such as 0.01% resulted in retardation of myopia progression, with significantly less side effects compared to higher concentration preparation.

The exact mechanism of topical atropine is still not known, although the up- and downregulation of retinal and scleral muscarinic receptors with influence on the scleral matrix have been postulated. Moreover, atropine inhibits myopia induction in both mammalian and avian eyes. Different to the mammalian eye, the avian eye contains striated ciliary muscle innervated by nicotinic receptor rather than muscarinic receptors. Therefore, atropine might have a function at a relatively lower dose, through M1/M4 receptors in the retina, not via the accommodation system. On the other hand, a non-muscarinic and a direct influence of atropine on the scleral fibroblasts could also contribute to the effect [Pei-Chang Wu, Meng-Ni Chuang, Update in myopia and treatment strategy of atropine use in myopia control, Eye (2019) 33:3-13].

Despite all this recent and very favourable clinical data, there is still currently no commercially available low dose atropine ophthalmic compositions.

### Ophthalmic composition

The preservative free ophthalmic low concentration atropine sulfate composition of the present invention is consisting of;
a) atropine sulfate in an amount from about 0.005 w/w% to about 0.05 w/w%
b) a tonicity agent
c) water
d) a pH adjuster, preferably hydrochloric acid
e) optionally a chelating agent, preferably EDTA
f) optionally a complexing agent, and
g) optionally a viscosity agent
characterized in that it has a pH from about 4.0 to about 5.5.

The amount of the atropine sulfate in the preservative free ophthalmic low concentration atropine sulfate composition according to the present invention is from about 0.005 w/w% to about 0.05 w/w%. Generally, the amount of atropine sulfate in the composition is from about 0.006 w/w% to about 0.05 w/w%, preferably is from about 0.007 w/w% to about 0.05 w/w%, preferably from about 0.008 w/w% to about 0.05 w/w%, preferably from about 0.009 w/w% to about 0.05 w/w%, more preferably from about 0.01 w/w% to about 0.05 w/w%. Still more preferably the amount of atropine sulfate in the composition is about 0.01 w/w% or about 0.05 w/w%. In the preservative free ophthalmic low concentration atropine sulfate composition according to the present invention the concentration of the atropine sulfate as w/w% is equivalent to w/v%.

The preservative free ophthalmic low concentration atropine sulfate composition has a pH from about 4.0 to about 5.5, preferably from about 4.0 to about 5.4, preferably from about 4.0 to about 5.3, preferably from about 4.0 to about 5.2, preferably from about 4.0 to about 5.1, preferably from about 4.0 to about 5.0, preferably from about 4.0 to about 4.9, preferably from about 4.0 to about 4.8, preferably from about 4.0 to about 4.7, preferably from about 4.0 to about 4.6, preferably from about 4.0 to about 4.5, preferably from about 4.0 to about 4.4, preferably from about 4.0 to about 4.3, preferably from about 4.0 to about 4.2, preferably from about 4.0 to about 4.1, preferably from about 4.1 to about 5.5, preferably from about 4.1 to about 5.4, preferably from about 4.1 to about 5.3, preferably from about 4.1 to about 5.2, preferably from about 4.1 to about 5.1, preferably from about 4.1 to about 5.0, preferably from about 4.1 to about 4.9, preferably from about 4.1 to about 4.8, preferably from about 4.1 to about 4.7, preferably from about 4.1 to about 4.6, preferably from about 4.1 to about 4.5, preferably from about 4.1 to about 4.4, preferably from about 4.1 to about 4.3, preferably from about 4.1 to about 4.2, preferably from about 4.2 to about 5.5, preferably from about 4.2 to about 5.4, preferably from about 4.2 to about 5.3, preferably from about 4.2 to about 5.2, preferably from about 4.2 to about 5.1, preferably from about 4.2 to about 5.0, preferably from about 4.2 to about 4.9, preferably from about 4.2 to about 4.8, preferably from about 4.2 to about 4.7, preferably from about 4.2 to about 4.6, preferably from about 4.2 to about 4.5, preferably from about 4.2 to about 4.4, preferably from about 4.2 to about 4.3, preferably from about 4.3 to about 5.5, preferably from about 4.3 to about 5.4, preferably from about 4.3 to about 5.3, preferably from about 4.3 to about 5.2, preferably from about 4.3 to about 5.1, preferably from about 4.3 to about 5.0, preferably from about 4.3 to about 4.9, preferably from about 4.3 to about 4.8, preferably from about 4.3 to about 4.7, preferably from about 4.3 to about 4.6, preferably from about 4.3 to about 4.5, preferably from about 4.3 to about 4.4, preferably from about 4.4 to about 5.5, preferably from about 4.4 to about 5.4, preferably from about 4.4 to about 5.3, preferably from about 4.4 to about 5.2, preferably from about 4.4 to about 5.1, preferably from about 4.4 to about 5.0, preferably from about 4.4 to about 4.9, preferably from about 4.4 to about 4.8, preferably from about 4.4 to about 4.7, preferably from about 4.4 to about 4.6, preferably from about 4.4 to about 4.5, preferably from about 4.5 to about 5.5, preferably from about 4.5 to about 5.4, preferably from about 4.5 to about 5.3, preferably from about 4.5 to about 5.2, preferably from about 4.5 to about 5.1, preferably from about 4.5 to about 5.0, preferably from about 4.5 to about 4.9, preferably from about 4.5 to about 4.8, preferably from about 4.5 to about 4.7, preferably from about 4.5 to about 4.6, more preferably is about 4.5. Still more preferably the preservative free ophthalmic low concentration atropine sulfate composition has a pH from about 4.0 to about 5.0, preferably from about 4.0 to 4.5, preferably about 4.5. In some embodiments the composition has a pH from about 4.0 to about 4.6, preferably from about 4.2 to about 4.6, preferably from about 4.4 to about 4.6. In specific pH case, the term "about" means to include the exact stated pH value and also a certain variation around such pH value, namely a variation of ±1% of the stated pH value.

The preservative free ophthalmic low concentration atropine sulfate composition according to the present invention also contains a tonicity agent. The term "tonicity agent" refers to a compound designed to reduce local irritation by preventing osmotic shock at the site of application. They are also known as osmotic pressure agents. Suitable tonicity agents include sodium chloride, potassium chloride, calcium chloride, glycerin, propylene glycol, glycerol, sorbitol, mannitol and the like. A preferred tonicity agent is sodium chloride. The amount of the tonicity agent in the preservative free ophthalmic low concentration atropine sulfate composition according to the present invention is from about 0.1 w/v% to about 2.0 w/v%. Preferably from about 0.5 w/v% to 1.0 w/v%, more preferably about 1.0 w/v% and more preferably about 0.9 w/v%.

The preservative free ophthalmic low concentration atropine sulfate composition according to the present invention also contains a pH adjuster. The term "pH adjuster" refers to a chemical compound which is used to alter pH of the aqueous solution within the range from about 4.0 to 5.5. When the pH needs to be lowered, the pH adjusting agent will be an inorganic acid such as hydrochloric acid or sulfuric acid. When the pH needs to be increased, the pH adjusting agent will be an inorganic base such as sodium hydroxide or potassium hydroxide. Preferred inorganic acid as pH adjuster is hydrochloric acid. Preferred inorganic base as pH adjuster is sodium hydroxide. The amount of the pH adjuster needed to adjust the pH of the composition to the desired range from about pH 4.0 to about 5.5 is significantly very low. This is the case specially when strong acid or base is used as pH adjuster. The amount of the pH adjuster in the ophthalmic composition is generally less than 0.001 w/v%, preferably less than 0.0001 w/v%, more preferably less than 0.00001 w/v%, still more preferably less than 0.0000001 w/v%.

Further, prior art ophthalmic low concentration atropine sulfate composition contains a buffer or mixtures of buffers to maintain the pH and the stability of the atropine solution. The term "buffer" refers to a substance capable in solution of neutralizing both acids and bases and thereby maintaining the original acidity or basicity of the solution. Examples of buffers suitable for ophthalmic compositions include but are not limited to boric acid, borax, citric acid, disodium hydrogenphosphate, ε-aminocaproic acid and the like. Surprisingly, the authors of the present invention have found that the preservative free ophthalmic low concentration atropine sulfate composition according to the present invention do not need a buffer to maintain the pH and the stability of the atropine solution. And also it guarantees the comfort of the eye upon instillation, as it allows the eye to adjust the pH to a physiological value of 7.4 as quick as possible.

The preservative free ophthalmic low concentration atropine sulfate composition according to the present invention also contains water.

As used herein, the term "ophthalmic composition" refers to liquid compositions that are suitable for topical ophthalmic delivery or eye drops, including solutions, suspensions and emulsions. In a specific embodiment the term "ophthalmic composition" refers to an ophthalmic solution. In a specific embodiment the term "ophthalmic composition" refers to an ophthalmic aqueous solution

Within this description, the "preservative-free ophthalmic solution" means that the ophthalmic solution of the present invention does not comprise a preservative, such as quaternary ammonium salts, e.g. benzalkonium chloride (BAK). Other pharmaceutically acceptable preservatives for ophthalmic solutions are for example boric acid-polyol-zinc chloride or chlorine oxide compounds, chlorhexidine gluconate, benzethonium chloride, sorbic acid, potassium sorbate, ethyl p-hydroxybenzoate and butyl p-hydroxybenzoate.

In a preferred embodiment, the preservative free ophthalmic low concentration atropine sulfate composition of the present invention is consisting of:
a) atropine sulfate in an amount from about 0.005 w/w% to about 0.05 w/w%
b) a tonicity agent
c) water, and
d) a pH adjuster, preferably hydrochloric acid
characterized in that it has a pH from about 4.0 to about 5.5.

In a preferred embodiment, the preservative free ophthalmic low concentration atropine sulfate composition of the present invention is consisting of:
a) atropine sulfate in an amount from about 0.005 w/w% to about 0.05 w/w%
b) a tonicity agent
c) water, and
d) a pH adjuster, preferably hydrochloric acid
characterized in that it has a pH from about 4.0 to about 5.0.

In a preferred embodiment, the preservative free ophthalmic low concentration atropine sulfate composition of the present invention is consisting of:
a) atropine sulfate in an amount from about 0.005 w/w% to about 0.05 w/w%
b) a tonicity agent
c) water, and
d) a pH adjuster, preferably hydrochloric acid
characterized in that it has a pH from about 4.0 to about 4.5.

In a preferred embodiment, the preservative free ophthalmic low concentration atropine sulfate composition of the present invention is consisting of:
a) atropine sulfate in an amount from about 0.005 w/w% to about 0.05 w/w%
b) a tonicity agent
c) water, and
d) a pH adjuster, preferably hydrochloric acid
characterized in that it has a pH about 4.5.

In a preferred embodiment, the preservative free ophthalmic low concentration atropine sulfate composition of the present invention is consisting of:
a) atropine sulfate in an amount from of about 0.01 w/w% or about 0.05 w/w%
b) a tonicity agent
c) water, and
d) a pH adjuster, preferably hydrochloric acid
characterized in that it has a pH from about 4.0 to about 5.5.

In a preferred embodiment, the preservative free ophthalmic low concentration atropine sulfate composition of the present invention is consisting of:
a) atropine sulfate in an amount of about 0.01 w/w% or about 0.05 w/w%
b) a tonicity agent
c) water, and
d) a pH adjuster, preferably hydrochloric acid
characterized in that it has a pH from about 4.0 to about 5.0.

In a preferred embodiment, the preservative free ophthalmic low concentration atropine sulfate composition of the present invention is consisting of:
a) atropine sulfate in an amount of about 0.01 w/w% or about 0.05 w/w%
b) a tonicity agent
c) water, and
d) a pH adjuster, preferably hydrochloric acid
characterized in that it has a pH from about 4.0 to about 4.5.

In a preferred embodiment, the preservative free ophthalmic low concentration atropine sulfate composition of the present invention is consisting of:
a) atropine sulfate in an amount of about 0.01 w/w% or about 0.05 w/w%
b) a tonicity agent
c) water, and
d) a pH adjuster, preferably hydrochloric acid
characterized in that it has a pH about 4.5.

The preservative free ophthalmic low concentration atropine sulfate composition according to the present invention may optionally have a chelating agent. As it is well-known in the field of pharmaceutical technology, chelating agents are commonly used to complex metal ions that can catalyze drug degradation They are also called chelants, chelators or sequestering agents. Suitable chelating agent to be used in this invention include, but are not limited to, ethylenediamine tetraacetic acid (EDTA), disodium edetate, calcium disodium edetate, trisodium edetate, tetrasodium edetate and/or combinations thereof. Preferably, the chelating agent is EDTA as a disodium salt. The amount of the chelating agent in the ophthalmic composition is generally from about 0.01% w/w to about 0.1% w/w, preferably 0.01% w/w. As described previously the presence of a chelating agent, preferably EDTA, in the preservative free ophthalmic low concentration atropine sulfate composition of the present invention assures the stability of the atropine in the composition that has been adjusted to pH from about 4.0 to about 5.5 without the presence of any buffer. Moreover, EDTA substantially increases the corneal permeability of atropine, thus improving the effectiveness of the drug.

In a preferred embodiment, the preservative free ophthalmic low concentration atropine sulfate composition of the present invention is consisting of:
a) atropine sulfate in an amount from about 0.005 w/w% to about 0.05 w/w%
b) a tonicity agent
c) water
d) a pH adjuster, preferably hydrochloric acid, and
e) optionally a chelating agent, preferably EDTA
characterized in that it has a pH from about 4.0 to about 5.5.

In a preferred embodiment, the preservative free ophthalmic low concentration atropine sulfate composition of the present invention is consisting of:
a) atropine sulfate in an amount from about 0.005 w/w% to about 0.05 w/w%
b) a tonicity agent
c) water, and
d) a pH adjuster, preferably hydrochloric acid, and
e) optionally a chelating agent, preferably EDTA
characterized in that it has a pH from about 4.0 to about 5.0.

In a preferred embodiment, the preservative free ophthalmic low concentration atropine sulfate composition of the present invention is consisting of:
a) atropine sulfate in an amount from about 0.005 w/w% to about 0.05 w/w%
b) a tonicity agent
c) water, and
d) a pH adjuster, preferably hydrochloric acid, and
e) optionally a chelating agent, preferably EDTA
characterized in that it has a pH from about 4.0 to about 4.5.

In a preferred embodiment, the preservative free ophthalmic low concentration atropine sulfate composition of the present invention is consisting of:
a) atropine sulfate in an amount from about 0.005 w/w% to about 0.05 w/w%
b) a tonicity agent
c) water, and
d) a pH adjuster, preferably hydrochloric acid, and
e) optionally a chelating agent, preferably EDTA
characterized in that it has a pH about 4.5.

In a preferred embodiment, the preservative free ophthalmic low concentration atropine sulfate composition of the present invention is consisting of:
a) atropine sulfate in an amount of about 0.01 w/w% or about 0.05 w/w%
b) a tonicity agent
c) water, and
d) a pH adjuster, preferably hydrochloric acid, and
e) optionally a chelating agent, preferably EDTA
characterized in that it has a pH from about 4.0 to about 5.5.

In a preferred embodiment, the preservative free ophthalmic low concentration atropine sulfate composition of the present invention is consisting of:
a) atropine sulfate in an amount of about 0.01 w/w% or about 0.05 w/w%
b) a tonicity agent
c) water, and
d) a pH adjuster, preferably hydrochloric acid, and
e) optionally a chelating agent, preferably EDTA
characterized in that it has a pH from about 4.0 to about 5.0.

In a preferred embodiment, the preservative free ophthalmic low concentration atropine sulfate composition of the present invention is consisting of:
a) atropine sulfate in an amount of about 0.01 w/w% or about 0.05 w/w%
b) a tonicity agent
c) water, and
d) a pH adjuster, preferably hydrochloric acid, and
e) optionally a chelating agent, preferably EDTA
characterized in that it has a pH from about 4.0 to about 4.5.

In a preferred embodiment, the preservative free ophthalmic low concentration atropine sulfate composition of the present invention is consisting of:
a) atropine sulfate in an amount of about 0.01 w/w% or about 0.05 w/w%
b) a tonicity agent
c) water, and
d) a pH adjuster, preferably hydrochloric acid, and
e) optionally a chelating agent, preferably EDTA
characterized in that it has a pH about 4.5.

The preservative free ophthalmic low concentration atropine sulfate composition according to the present invention may optionally have a complexing agent. Complexing agents are compounds, which can form relatively stable complexes with drugs by somewhat strong coordinate covalent bonds or by relatively weak noncovalent forces such as hydrogen bonds, van der Waals forces, electrostatic interactions, dipole forces or hydrophobic interactions. In the ophthalmic products complexing agent are mainly used to increase the aqueous solubility of poorly water-soluble drugs, to increase their bioavailability and stability as well as reduce the ocular irritation or prevent drug-drug or drug-additive interactions. Said complexing agents are typically cyclodextrins, as are well for the skilled person in pharmaceutical composition. Suitable cyclodextrins to be used in the compositions of this invention are β-cyclodextrin, γ- cyclodextrin, hydroxypropyl-β-cyclodextrin, sulfobutylether β-cyclodextrin Preferably, the complexing agent is hydroxypropyl-β-cyclodextrin. The amount of the complexing agent in the ophthalmic composition is generally from about 0.001% w/w to about 1% w/w, preferably from about 0.005% w/w to about 0.2% w/w, more preferably from about 0.01% w/w to about 0.1% w/w, more preferably from about 0.015% w/w to about 0.05% w/w, more preferably from about 0.02% w/w to about 0.025% w/w,and more preferably about 0.022% w/w. It has been found that the further presence of complexing agent allows to form an inclusion complex with atropine molecule, which increases its long-term stability.

In a preferred embodiment, the preservative free ophthalmic low concentration atropine sulfate composition of the present invention is consisting of:
a) atropine sulfate in an amount from about 0.005 w/w% to about 0.05 w/w%
b) sodium chloride as tonicity agent
c) water
d) a pH adjuster, preferably hydrochloric acid
e) optionally a chelating agent, preferably EDTA, and
f) optionally a complexing agent, preferably hydroxypropyl-P-cyclodextrin
characterized in that it has a pH from about 4.0 to about 5.5.

In a preferred embodiment, the preservative free ophthalmic low concentration atropine sulfate composition of the present invention is consisting of:
a) atropine sulfate in an amount from about 0.005 w/w% to about 0.05 w/w%
b) sodium chloride as tonicity agent
c) water
d) a pH adjuster, preferably hydrochloric acid
e) optionally a chelating agent, preferably EDTA, and
f) optionally a complexing agent, preferably hydroxypropyl-β-cyclodextrin
characterized in that it has a pH from about 4.0 to about 5.0.

In a preferred embodiment, the preservative free ophthalmic low concentration atropine sulfate composition of the present invention is consisting of:
a) atropine sulfate in an amount from about 0.005 w/w% to about 0.05 w/w%
b) sodium chloride as tonicity agent
c) water
d) a pH adjuster, preferably hydrochloric acid
e) optionally a chelating agent, preferably EDTA, and
f) optionally a complexing agent, preferably hydroxypropyl-β-cyclodextrin
characterized in that it has a pH from about 4.0 to about 4.5.

In a preferred embodiment, the preservative free ophthalmic low concentration atropine sulfate composition of the present invention is consisting of:
a) atropine sulfate in an amount from about 0.005 w/w% to about 0.05 w/w%
b) sodium chloride as tonicity agent
c) water
d) a pH adjuster, preferably hydrochloric acid
e) optionally a chelating agent, preferably EDTA, and
f) optionally a complexing agent, preferably hydroxypropyl-p-cyclodextrin
characterized in that it has a pH about 4.5.

In a preferred embodiment, the preservative free ophthalmic low concentration atropine sulfate composition of the present invention is consisting of:
a) atropine sulfate in an amount of about 0.01 w/w% or about 0.05 w/w%
b) sodium chloride as tonicity agent
c) water
d) a pH adjuster, preferably hydrochloric acid
e) optionally a chelating agent, preferably EDTA, and
f) optionally a complexing agent, preferably hydroxypropyl-β-cyclodextrin
characterized in that it has a pH from about 4.0 to about 5.5.

In a preferred embodiment, the preservative free ophthalmic low concentration atropine sulfate composition of the present invention is consisting of:
a) atropine sulfate in an amount of about 0.01 w/w% or about 0.05 w/w%
b) sodium chloride as tonicity agent
c) water
d) a pH adjuster, preferably hydrochloric acid
e) optionally a chelating agent, preferably EDTA, and
f) optionally a complexing agent, preferably hydroxypropyl-β-cyclodextrin
characterized in that it has a pH from about 4.0 to about 5.0.

In a preferred embodiment, the preservative free ophthalmic low concentration atropine sulfate composition of the present invention is consisting of:
a) atropine sulfate in an amount of about 0.01 w/w% or about 0.05 w/w%
b) sodium chloride as tonicity agent
c) water
d) a pH adjuster, preferably hydrochloric acid
e) optionally a chelating agent, preferably EDTA, and
f) optionally a complexing agent, preferably hydroxypropyl-β-cyclodextrin
characterized in that it has a pH from about 4.0 to about 4.5.

In a preferred embodiment, the preservative free ophthalmic low concentration atropine sulfate composition of the present invention is consisting of:
a) atropine sulfate in an amount of about 0.01 w/w% or about 0.05 w/w%
b) sodium chloride as tonicity agent
c) water
d) a pH adjuster, preferably hydrochloric acid
e) optionally a chelating agent, preferably EDTA, and
f) optionally a complexing agent, preferably hydroxypropyl-β-cyclodextrin
characterized in that it has a pH about 4.5.

The preservative free ophthalmic low concentration atropine sulfate composition according to the present invention may optionally have a viscosity agent. A viscosity agent is a compound which significantly increase the viscosity of the formulation. In ocular drug delivery systems, a viscosity agent prolongs corneal contact time and, thus, improves drug bioavailability. Said viscosity agents are typically pharmaceutically acceptable polymers, as are well known for the skilled person in pharmaceutical formulation. Suitable pharmaceutically acceptable polymers include, for example, microcrystalline cellulose, hydroxypropyl methylcellulose (Hypromellose, HPMC), hydroxyethyl cellulose (HEC), carboxy methylcellulose, polyvinyl alcohol (PVA), polyethylene glycol (PEG) or natural polymers, such as hyaluronic acid, guar gum, gellan gum and the mixture thereof. Preferably, the pharmaceutically acceptable polymers are hydroxypropyl methylcellulose and hydroxyethyl cellulose. More preferably, hydroxyethyl cellulose. The amount of the pharmaceutically polymer in the ophthalmic composition is generally from about 0.01 to about 0,5% w/v, preferably from about 0.1% w/v to about 0,35% w/v, more preferably from about 0,15% w/v to about 0.25% w/v.

In a preferred embodiment, the preservative free ophthalmic low concentration atropine sulfate composition of the present invention is consisting of:
a) atropine sulfate in an amount from about 0.005 w/w% to about 0.05 w/w%
b) sodium chloride as tonicity agent
c) water
d) a pH adjuster, preferably hydrochloric acid
e) optionally a chelating agent, preferably EDTA
f) optionally a complexing agent, preferably hydroxypropyl-P-cyclodextrin, and
g) optionally a viscosity agent, preferably hydroxyethyl cellulose
characterized in that it has a pH from about 4.0 to about 5.5.

In a preferred embodiment, the preservative free ophthalmic low concentration atropine sulfate composition of the present invention is consisting of:
a) atropine sulfate in an amount from about 0.005 w/w% to about 0.05 w/w%
b) sodium chloride as tonicity agent
c) water
d) a pH adjuster, preferably hydrochloric acid
e) optionally a chelating agent, preferably EDTA
f) optionally a complexing agent, preferably hydroxypropyl-β-cyclodextrin, and
g) optionally a viscosity agent, preferably hydroxyethyl cellulose
characterized in that it has a pH from about 4.0 to about 5.0.

In a preferred embodiment, the preservative free ophthalmic low concentration atropine sulfate composition of the present invention is consisting of:
a) atropine sulfate in an amount from about 0.005 w/w% to about 0.05 w/w%
b) sodium chloride as tonicity agent
c) water
d) a pH adjuster, preferably hydrochloric acid
e) optionally a chelating agent, preferably EDTA
f) optionally a complexing agent, preferably hydroxypropyl-β-cyclodextrin, and
g) optionally a viscosity agent, preferably hydroxyethyl cellulose
characterized in that it has a pH from about 4.0 to about 4.5.

In a preferred embodiment, the preservative free ophthalmic low concentration atropine sulfate composition of the present invention is consisting of:
a) atropine sulfate in an amount from about 0.005 w/w% to about 0.05 w/w%
b) sodium chloride as tonicity agent
c) water
d) a pH adjuster, preferably hydrochloric acid
e) optionally a chelating agent, preferably EDTA
f) optionally a complexing agent, preferably hydroxypropyl-P-cyclodextrin, and
g) optionally a viscosity agent, preferably hydroxyethyl cellulose
characterized in that it has a pH about 4.5.

In a preferred embodiment, the preservative free ophthalmic low concentration atropine sulfate composition of the present invention is consisting of:
a) atropine sulfate in an amount of about 0.01 w/w% or about 0.05 w/w%
b) sodium chloride as tonicity agent
c) water
d) a pH adjuster, preferably hydrochloric acid
e) optionally a chelating agent, preferably EDTA
f) optionally a complexing agent, preferably hydroxypropyl-β-cyclodextrin, and
g) optionally a viscosity agent, preferably hydroxyethyl cellulose
characterized in that it has a pH from about 4.0 to about 5.5.

In a preferred embodiment, the preservative free ophthalmic low concentration atropine sulfate composition of the present invention is consisting of:
a) atropine sulfate in an amount of about 0.01 w/w% or about 0.05 w/w%
b) sodium chloride as tonicity agent
c) water
d) a pH adjuster, preferably hydrochloric acid
e) optionally a chelating agent, preferably EDTA
f) optionally a complexing agent, preferably hydroxypropyl-P-cyclodextrin, and
g) optionally a viscosity agent, preferably hydroxyethyl cellulose
characterized in that it has a pH from about 4.0 to about 5.0.

In a preferred embodiment, the preservative free ophthalmic low concentration atropine sulfate composition of the present invention is consisting of:
a) atropine sulfate in an amount of about 0.01 w/w% or about 0.05 w/w%
b) sodium chloride as tonicity agent
c) water
d) a pH adjuster, preferably hydrochloric acid
e) optionally a chelating agent, preferably EDTA
f) optionally a complexing agent, preferably hydroxypropyl-β-cyclodextrin, and
g) optionally a viscosity agent, preferably hydroxyethyl cellulose
characterized in that it has a pH from about 4.0 to about 4.5.

In a preferred embodiment, the preservative free ophthalmic low concentration atropine sulfate composition of the present invention is consisting of:
a) atropine sulfate in an amount of about 0.01 w/w% or about 0.05 w/w%
b) sodium chloride as tonicity agent
c) water
d) a pH adjuster, preferably hydrochloric acid
e) optionally a chelating agent, preferably EDTA
f) optionally a complexing agent, preferably hydroxypropyl-β-cyclodextrin, and
g) optionally a viscosity agent, preferably hydroxyethyl cellulose
characterized in that it has a pH about 4.5.

### Dosage form

The pharmaceutical dosage form of the present invention is an ophthalmic composition is the form of an aqueous solution.

Typically, the aqueous solution is filled container. A container is referred herein also as a bottle, reservoir or vessel. Typically, containers for ophthalmic compositions are made of a polymer. Suitable polymers include, but are not limited to, polyethylene (PE), polypropylene (PP), polyethylene terephthalate (PET), polyvinyl chloride, acrylic resins, polystyrene, polymethylmethacrylate, nylon 6 and mixtures thereof. In a preferred embodiment, the container is made of polyethylene (PE) or polypropylene (PP). These polymers can be high-density or low-density polymers, e.g high density polyethylene.

Containers made of PP as well as PE are shatterproof and lightweight. These materials are well known and used for decades. In relation to the extractables they are considered safe and neutral to the packaging content.

Said containers for the aqueous solution can be manufactured by both an injection-blow moulding and a blow-fill-seal processes, which are widely known in pharmaceuticals industry. In a preferred embodiment, the container is manufactured by the injection-blow moulding process.

Typically, the containers suitable for ophthalmic compositions have a dropper system. The preservative free ophthalmic low concentration atropine sulfate composition of the present invention refers to a multidose use of the composition. Typically, ophthalmic compositions suitable for multidose use have to guarantee the sterility of the solution after first opening of the device and during following uses. Preservatives are commonly added to guarantee this sterility. However, they can be toxic and pose problems of tolerance, especially in the context of a long-term treatment such as myopia. In preservative free ophthalmic compositions of the present invention, the sterility is guaranteed with specific dropper systems, which are tightly attached to the container. Suitable dropper systems for a multidose use of the preservative free ophthalmic solutions may have a special pump or require a bottle squeezing to open a valve and deliver the drop with an exact volume. In a preferred embodiment, the dropper system is equipped with the pump.

### Preparation of the dosage form

Another aspect of the present invention is a process for the preparation of the preservative free ophthalmic low concentration atropine sulfate composition, as defined above, comprising the following steps:
i) dissolve all the components except atropine sulfate in water
ii) dissolve the atropine sulfate in the solution obtained in step i)
iii) sterile filtration of the solution obtained in step ii)
iv) dispense the filtered solution obtained in step iii) to a sterile container
v) aseptic closure of the container obtained in step iv) by a sterile dropper or dropper system suitable for ophthalmic compositions

### Use of the dosage form

As shown in the stability test disclosed in Examples 1A, 1B, 1C and 1C, surprisingly, the dosage form according to the present invention is outstandingly stable and is therefore particularly suitable for myopia.

Furthermore, as shown in Example 1A, 1B, 1C and 1D, the dosage form of the invention has particularly low levels of tropic acid impurities.

Then, the present dosage form is advantageous in terms of safety, over the prior art solutions, due to the lack of buffer used in the composition, as the possible adverse events related to some buffers used for ophthalmic solutions are minimized. Furthermore, the present dosage form comprising the ophthalmic composition of the present invention has also the advantage of eye comfort, as it allows the eye to adjust the pH to a physiological value of 7.4 as quick as possible.

Therefore, another aspect of the invention is the preservative free ophthalmic low concentration atropine sulfate composition according to the present invention, or the pharmaceutical dosage form comprising it, for use in therapy, preferably, for use in the treatment and/or prevention of myopia, preferably in paediatric population.

Another aspect of the present invention is a method for treating and/or preventing myopia in a patient in need thereof, preferably in paediatric population, comprising the step of administering therapeutically effective amount of the preservative free ophthalmic low concentration atropine sulfate composition or the dosage form of the present invention.

The present invention relates to the following embodiments:
A preservative free ophthalmic low concentration atropine sulfate composition consisting of:
a) atropine sulfate in an amount from about 0.005 w/w% to about 0.05 w/w%, preferably about 0.01 w/w% or about 0.05 w/w%
b) a tonicity agent
c) water
d) a pH adjuster
e) optionally a chelating agent
f) optionally a complexing agent, and
g) optionally a viscosity agent
characterized in that it has a pH from about 4.0 to about 5.5, preferably from about 4.0 to about 5.0, preferably from about 4.1 to about 4.5, preferably from about 4.1 to about 4.9, preferably from about 4.2 to about 4.8, preferably from about 4.3 to about 4.7, preferably from about 4.4 to about 4.6, more preferably about 4.5.

A preservative free ophthalmic low concentration atropine sulfate composition consisting of:
a) atropine sulfate in an amount from about 0.005 w/w% to about 0.05 w/w%, preferably about 0.01 w/w% or about 0.05 w/w%
b) sodium chloride as tonicity agent
c) water
d) hydrochloric acid as pH adjuster
e) optionally a chelating agent
f) optionally a complexing agent, and
g) optionally a viscosity agent
characterized in that it has a pH from about 4.0 to about 5.5, preferably from about 4.0 to about 5.0, preferably from about 4.1 to about 4.5, preferably from about 4.1 to about 4.9, preferably from about 4.2 to about 4.8, preferably from about 4.3 to about 4.7, preferably from about 4.4 to about 4.6, more preferably about 4.5.

A preservative free ophthalmic low concentration atropine sulfate composition consisting of:
a) atropine sulfate in an amount from about 0.005 w/w% to about 0.05 w/w%, preferably about 0.01 w/w% or about 0.05 w/w%
b) sodium chloride as tonicity agent
c) water, and
d) hydrochloric acid as pH adjuster
characterized in that it has a pH from about 4.0 to about 5.5, preferably from about 4.0 to about 5.0, preferably from about 4.1 to about 4.5, preferably from about 4.1 to about 4.9, preferably from about 4.2 to about 4.8, preferably from about 4.3 to about 4.7, preferably from about 4.4 to about 4.6, more preferably about 4.5.

A preservative free ophthalmic low concentration atropine sulfate composition consisting of:
a) atropine sulfate in an amount from about 0.005 w/w% to about 0.05 w/w%, preferably about 0.01 w/w% or about 0.05 w/w%
b) sodium chloride as tonicity agent
c) water, and
d) hydrochloric acid as pH adjuster
characterized in that it has a pH about 4.5.

A preservative free ophthalmic low concentration atropine sulfate composition consisting of:
a) atropine sulfate in an amount of about 0.01 w/w% or about 0.05 w/w%
b) sodium chloride as tonicity agent
c) water, and
d) hydrochloric acid as pH adjuster
characterized in that it has a pH about 4.5.

A preservative free ophthalmic low concentration atropine sulfate composition consisting of:
a) atropine sulfate in an amount from about 0.005 w/w% to about 0.05 w/w%, preferably about 0.01 w/w% or about 0.05 w/w%
b) sodium chloride as tonicity agent
c) water
d) hydrochloride acid as pH adjuster
e) EDTA as chelating agent
f) optionally a complexing agent, and
g) optionally a viscosity agent
characterized in that it has a pH from about 4.0 to about 5.5, preferably from about 4.0 to about 5.0, preferably from about 4.1 to about 4.5, preferably from about 4.1 to about 4.9, preferably from about 4.2 to about 4.8, preferably from about 4.3 to about 4.7, preferably from about 4.4 to about 4.6, more preferably about 4.5.

A preservative free ophthalmic low concentration atropine sulfate composition consisting of:
a) atropine sulfate in an amount from about 0.005 w/w% to about 0.05 w/w%, preferably about 0.01 w/w% or about 0.05 w/w%
b) sodium chloride as tonicity agent
c) water
d) hydrochloride acid as pH adjuster, and
e) EDTA as chelating agent
characterized in that it has a pH from about 4.0 to about 5.5, preferably from about 4.0 to about 5.0, preferably from about 4.1 to about 4.5, preferably from about 4.1 to about 4.9, preferably from about 4.2 to about 4.8, preferably from about 4.3 to about 4.7, preferably from about 4.4 to about 4.6, more preferably about 4.5.

A preservative free ophthalmic low concentration atropine sulfate composition consisting of:
a) atropine sulfate in an amount from about 0.005 w/w% to about 0.05 w/w%, preferably about 0.01 w/w% or about 0.05 w/w%
b) sodium chloride as tonicity agent
c) water
d) hydrochloride acid as pH adjuster, and
e) EDTA as chelating agent
characterized in that it has a pH from about about 4.5.

A preservative free ophthalmic low concentration atropine sulfate composition consisting of:
a) atropine sulfate in an amount of about 0.01 w/w% or about 0.05 w/w%
b) sodium chloride as tonicity agent
c) water
d) hydrochloride acid as pH adjuster, and
e) EDTA as chelating agent
characterized in that it has a pH from about about 4.5.

A preservative free ophthalmic low concentration atropine sulfate composition consisting of:
a) atropine sulfate in an amount from about 0.005 w/w% to about 0.05 w/w%, preferably about 0.01 w/w% or about 0.05 w/w%
b) sodium chloride as tonicity agent
c) water
d) hydrochloride acid as pH adjuster
e) EDTA as chelating agent, and
f) optionally a complexing agent, preferably hydroxypropyl-p-cyclodextrin
characterized in that it has a pH from about 4.0 to about 5.5, preferably from about 4.0 to about 5.0, preferably from about 4.1 to about 4.5, preferably from about 4.1 to about 4.9, preferably from about 4.2 to about 4.8, preferably from about 4.3 to about 4.7, preferably from about 4.4 to about 4.6, more preferably about 4.5.

A preservative free ophthalmic low concentration atropine sulfate composition consisting of:
a) atropine sulfate in an amount from about 0.005 w/w% to about 0.05 w/w%, preferably about 0.01 w/w% or about 0.05 w/w%
b) sodium chloride as tonicity agent
c) water
d) hydrochloride acid as pH adjuster
e) EDTA as chelating agent, and
f) hydroxypropyl-β-cyclodextrin as complexing agent
characterized in that it has a pH about 4.5.

A preservative free ophthalmic low concentration atropine sulfate composition consisting of:
a) atropine sulfate in an amount of about 0.01 w/w% or about 0.05 w/w%
b) sodium chloride as tonicity agent
c) water
d) hydrochloride acid as pH adjuster
e) EDTA as chelating agent, and
f) hydroxypropyl-β-cyclodextrin as complexing agent
characterized in that it has a pH about 4.5.

A preservative free ophthalmic low concentration atropine sulfate composition consisting of:
a) atropine sulfate in an amount from about 0.005 w/w% to about 0.05 w/w%, preferably about 0.01 w/w% or about 0.05 w/w%
b) sodium chloride as tonicity agent
c) water
d) hydrochloride acid as pH adjuster
e) EDTA as chelating agent, and
f) optionally a viscosity agent, preferably hydroxyethyl cellulose
characterized in that it has a pH from about 4.0 to about 5.5, preferably from about 4.0 to about 5.0, preferably from about 4.1 to about 4.5, preferably from about 4.1 to about 4.9, preferably from about 4.2 to about 4.8, preferably from about 4.3 to about 4.7, preferably from about 4.4 to about 4.6, more preferably about 4.5.

A preservative free ophthalmic low concentration atropine sulfate composition consisting of:
a) atropine sulfate in an amount from about 0.005 w/w% to about 0.05 w/w%, preferably about 0.01 w/w% or about 0.05 w/w%
b) sodium chloride as tonicity agent
c) water
d) hydrochloride acid as pH adjuster
e) EDTA as chelating agent, and
f) hydroxyethyl cellulose as viscosity agent
characterized in that it has a pH about 4.5.

A preservative free ophthalmic low concentration atropine sulfate composition consisting of:
a) atropine sulfate in an amount from of about 0.01 w/w% or about 0.05 w/w%
b) sodium chloride as tonicity agent
c) water
d) hydrochloride acid as pH adjuster
e) EDTA as chelating agent, and
f) hydroxyethyl cellulose as viscosity agent
characterized in that it has a pH about 4.5.

A process for the preparation of the preservative free ophthalmic low concentration atropine sulfate composition according to any one of claims 1 to 11, characterized in that it comprises the following steps:
i) dissolving all the components except atropine sulfate in water
ii) dissolving the atropine sulfate in the solution obtained in step i)
iii) sterile filtration of the solution obtained in step ii)
iv) dispense the filtered solution obtained in step iii) to a sterile container
v) aseptic closing of the container obtained in step iv) by the special dropper

### Examples

### Example 1. Ophthalmic preservative free low concentration atropine sulfate composition of the present invention

Solutions 1A, 1B, 1C and 1D comprising 0.01% w/w of atropine sulfate were prepared using the ingredients of Table 1:

**TABLE 1**

| **Ingredient** | **Example 1A** | **Example 1B** | **Example 1C** | **Example 1D** |
|---|---|---|---|---|
| | **% w/w** | **% w/w** | **% w/w** | **% w/w** |
| Atropine sulfate | 0.01 | 0.01 | 0.01 | 0.01 |
| Sodium chloride | 0.9 | 0.9 | 0.9 | 0.9 |
| Disodium edetate | - | 0.01 | 0.01 | 0.01 |
| Hydrochloric acid | q.s. | q.s. | q.s. | q.s. |
| Water for injections | Up to 1 mL | Up to 1 mL | Up to 1 mL | Up to 1 mL |
| hydroxypropyl-β-cyclodextrin | -- | -- | 0.02 | --- |
| hydroxyethyl cellulose | -- | -- | -- | 0.25 |
| pH | 4.5 | 4.5 | 4.5 | 4.5 |

The preservative free ophthalmic low concentration atropine sulfate compositions of Examples 1A, 1B and 1C were prepared by dissolving the excipients in 2/3 water. Then atropine sulfate was added and after complete dissolution the rest of the water was added. The pH was checked and the pH adjuster solution was added dropwise to adjust the pH. Next, the sterile filtration through 0.2 microns filter was carried out. The sterile solution was filled in sterilized HPDE bottles, and the bottles were tightly closed by the dropper system with a special pump. In case of Example 1D the sterile filtration through 0.2 microns filter was not carried out. Finished dosage form was storage at temperature below 25°C.

The pharmaceutical compositions of Table 1 were subjected to accelerated stability studies at 40 °C ± 2 °C / 75% ± 5% RH as per the International Committee on Harmonization stability conditions for 2 months. The results are shown in Tables 2-5.

**TABLE 2**

| **Example 1A - 40 °C ± 2 °C /75% ± 5% RH** | | | |
|---|---|---|---|
| | initial | 1 month | 2 months |
| pH | 4.50 | 4.30 | 4.20 |
| Osmolarity / mOsm/kg | 287 | 287 | 285 |
| assay of atropine sulfate / % of w/v | 101.8 | 100.3 | 99.1 |
| Impurity C (tropic acid) / % of w/v | 0.00 | 0.00 | 0.15 |
| Total impurities / % of w/v | 0.00 | 0.00 | 0.15 |

**TABLE 3**

| **Example 1B - 40 °C ± 2 °C /75% ± 5% RH** | | | |
|---|---|---|---|
| | initial | 1 month | 2 months |
| pH | 4.60 | 4.40 | 4.40 |
| Osmolarity / mOsm/kg | 287 | 287 | 287 |
| assay of atropine sulfate / % of w/v | 101.5 | 100.0 | 99.5 |
| Impurity C (tropic acid) / % of w/v | 0.00 | 0.15 | 0.25 |
| Total impurities / % of w/v | 0.00 | 0.15 | 0.25 |

**TABLE 4**

| **Example 1C - 40 °C ± 2 °C / 75% ± 5% RH** | | | |
|---|---|---|---|
| | initial | 1 month | 2 months |
| pH | 4.50 | 4.40 | 4.20 |
| Osmolarity / mOsm/kg | 289 | 287 | 283 |
| assay of atropine sulfate / % of w/v | 102.0 | 100.7 | 99.7 |
| Impurity C (tropic acid) / % of w/v | 0.00 | 0.00 | 0.10 |
| Total impurities / % of w/v | 0.00 | 0.00 | 0.10 |

**TABLE 5**

| **Example 1D - 40 °C ± 2 °C / 75% ± 5% RH** | | | |
|---|---|---|---|
| | initial | 1 month | 2 months |
| pH | 4.50 | 4.40 | 4.20 |
| Osmolarity / mOsm/kg | 289 | 287 | 283 |
| assay of atropine sulfate / % of w/v | 102.0 | 100.7 | 99.7 |
| Impurity C (tropic acid) / % of w/v | 0.00 | 0.00 | 0.00 |
| Total impurities / % of w/v | 0.00 | 0.00 | 0.00 |

### COMPARATIVE EXAMPLES

Solutions 2A and 2B were prepared analogously as disclosed in example 1, using the ingredients of Table 6.

**TABLE 6**

| **Ingredient** | **Example 2A** | **Example 2B** |
|---|---|---|
| | **% w/w** | **% w/w** |
| Atropine sulfate | 0.01 | 0.01 |
| Sodium chloride | 0.9 | 0.9 |
| Disodium edetate | - | - |
| Hydrochloric acid | - | q.s. |
| Water for injections | Up to 1 mL | Up to 1 mL |
| pH | 5.8 | 5.6 |

The pharmaceutical compositions of Table 6 were subjected to accelerated stability studies at 40 °C ± 2 °C / 75% ± 5% RH as per the International Committee on Harmonization stability conditions for 3 months. Samples were measured after 1 month and 2 and 3 months. The results are shown in Tables 7-8.

**TABLE 7**

| **Example 2A - 40 °C ± 2 °C / 75% ± 5% RH** | | | | |
|---|---|---|---|---|
| | initial | 1 month | 2 months | 3 months |
| pH | 5.8 | 5.40 | 5.20 | 4.90 |
| Osmolarity / mOsm/kg | 290 | 286 | 288 | 293 |
| Assay of atropine sulfate / % of w/w | 102.7 | 100.8 | 99.3 | 98.3 |
| Impurity C (tropic acid) / % of w/v | 0.00 | 0.94 | 1.45 | 1.95 |
| Total impurities / % of w/v | 0.27 | 1.05 | 1.64 | 2.17 |

**TABLE 8**

| **Example 2B - 40 °C ± 2 °C / 75% ± 5% RH** | | | | |
|---|---|---|---|---|
| | initial | 1 month | 2 months | 3 months |
| pH | 5.6 | 5.30 | 5.20 | 5.10 |
| Osmolarity / mOsm/kg | 291 | 281 | 285 | 287 |
| Assay of atropine sulfate / % of w/w | 102.5 | 101.9 | 99.3 | 98.8 |
| Impurity C (tropic acid) / % of w/v | 0.00 | 0.92 | 1.39 | 1.59 |
| Total impurities / % of w/v | 0.25 | 1.19 | 1.60 | 1.81 |

Comparative Examples 2A and 2B demonstrated greater instability in comparison to solutions of Example 1. While osmolarity remained relatively unchanged and within acceptable levels, the fluctuations of pH values can be observed through the study durations. Moreover, an almost twenty times higher level of total impurities after three months at 40 °C/75%RH than in solutions of Example 1 were noticeable.

## Claims

1. A preservative free ophthalmic low concentration atropine sulfate composition consisting of:
a) atropine sulfate in an amount from about 0.005 w/w% to about 0.05 w/w%
b) a tonicity agent
c) water
d) a pH adjuster
e) optionally a chelating agent
f) optionally a complexing agent, and
g) optionally a viscosity agent
**characterized in that** it has a pH from about 4.0 to about 5.5.

2. The preservative free ophthalmic low concentration atropine sulfate composition according to claim 1, **characterized in that** the pH is from about 4.0 to about 5.0, preferably from about 4.1 to about 4.5, preferably from about 4.1 to about 4.9, preferably from about 4.2 to about 4.8, preferably from about 4.3 to about 4.7, preferably from about 4.4 to about 4.6, more preferably about 4.5.

3. The preservative free ophthalmic low concentration atropine sulfate composition according to any one of claims 1 to 2, **characterized in that** the pH adjuster is hydrochloric acid.

4. The preservative free ophthalmic low concentration atropine sulfate composition according to any one of claims 1 to 3, **characterized in that** the chelating agent is EDTA.

5. The preservative free ophthalmic low concentration atropine sulfate composition according to any one of claims 1 to 4, **characterized in that** the tonicity agent is sodium chloride.

6. The preservative free ophthalmic low concentration atropine sulfate composition according to any one of claims 1 to 5, **characterized in that** the complexing agent is cyclodextrin compound, preferably β-cydodextrin, γ- cyclodextrin, HP-β- cyclodextrin or sulfobutylether β-cydodextrin, more preferably hydroxypropyl-β- cyclodextrin.

7. The preservative free ophthalmic low concentration atropine sulfate composition according to any one of claims 1 to 6, **characterized in that** amount of atropine sulfate is 0.01 w/w% or 0.05 w/w%.

8. The preservative free ophthalmic low concentration atropine sulfate composition according to any one of claims 1 to 7 consisting of:
a) atropine sulfate in an amount of about 0.01 w/w% or about 0.05 w/w%
b) sodium chloride as tonicity agent
c) water
d) hydrochloric acid as pH adjuster, and
e) optionally a chelating agent, preferably EDTA
**characterized in that** it has a pH from about 4.0 to about 5.5.

9. The preservative free ophthalmic low concentration atropine sulfate composition according to any one of claims 1 to 8 consisting of:
a) atropine sulfate in an amount of about 0.01 w/w% or about 0.05 w/w%
b) sodium chloride as tonicity agent
c) water
d) hydrochloric acid as pH adjuster, and
e) optionally a chelating agent, preferably EDTA
**characterized in that** it has a pH about 4.5.

10. The preservative free ophthalmic low concentration atropine sulfate composition according to any one of claims 1 to 9 consisting of:
a) atropine sulfate in an amount of about 0.01 w/w% or about 0.05 w/w%
b) sodium chloride as tonicity agent
c) water
d) hydrochloric acid as pH adjuster, and
e) EDTA as chelating agent
**characterized in that** it has a pH from about 4.0 to about 5.5.

11. The preservative free ophthalmic low concentration atropine sulfate composition according to any of claims 1 to 10 consisting of:
a) atropine sulfate in an amount of about 0.01 w/w% or about 0.05 w/w%
b) sodium chloride as tonicity agent
c) water
d) hydrochloric acid as pH adjuster, and
e) EDTA as chelating agent
**characterized in that** it has a pH about 4.5.

12. A process for the preparation of the preservative free ophthalmic low concentration atropine sulfate composition according to any one of claims 1 to 11, **characterized in that** it comprises the following steps:
i) dissolving all the components except atropine sulfate in water
ii) dissolving the atropine sulfate in the solution obtained in step i)
iii) sterile filtration of the solution obtained in step ii)
iv) dispense the filtered solution obtained in step iii) to a sterile container
v) aseptic closing of the container obtained in step iv) by the special dropper

13. A pharmaceutical dosage form comprising preservative free ophthalmic low concentration atropine sulfate composition according to any one of claims 1 to 11, preferably in the form of a solution.

14. The preservative free ophthalmic low concentration atropine sulfate composition according to any one of claims 1 to 11 or the pharmaceutical dosage form according to claim 13 for use in therapy, preferably, for use in the treatment and/or prevention of myopia, preferably in a paediatric population.

15. A method for treating and/or preventing myopia in a patient in need thereof, preferably in a paediatric population, comprising the step of administering therapeutically effective amount of the preservative free ophthalmic low concentration atropine sulfate composition according to any one of claims 1 to 11 or the pharmaceutical dosage form according to claim 13.
